# EUROPÄISCHE PATENTSCHRIFT

(11) **EP 2 560 728 B1**
(45) Veröffentlichungstag und Bekanntmachung des Hinweises auf die Patenterteilung: **20.04.2016**
(21) Anmeldenummer: 12724563.7
(22) Anmeldetag: 16.02.2012
(51) Int. Cl.: A61M 39/10

(54) **VERBINDUNGSSYSTEM FÜR LEITUNGEN IM MEDIZINISCHEN BEREICH**
CONNECTING SYSTEM FOR LINES IN THE MEDICAL SECTOR
SYSTÈME DE RACCORDEMENT DE CONDUITES DANS LE DOMAINE MÉDICAL

(30) Priorität: 18.02.2011 DE 102011011762
(43) Veröffentlichungstag der Anmeldung: 27.02.2013
(73) Patentinhaber: Geuder AG, 69126 Heidelberg (DE)
(72) Erfinder: DRAHEIM, René, 69207 Sandhausen (DE); ENGEL, Stefan, 69124 Heidelberg (DE)
(74) Vertreter: Patent- und Rechtsanwälte Ullrich & Naumann
(86) Internationale Anmeldenummer: PCT/DE2012/200010
(87) Internationale Veröffentlichungsnummer: WO 2012/110035

(56) Entgegenhaltungen:
- EP-A2- 1 902 747
- WO-A1-2008/009946
- FR-A1- 2 924 616
- GB-A- 2 451 891
- US-A1- 2006 033 331
- US-A1- 2008 318 456

## Beschreibung

Die vorliegende Erfindung betrifft einen Luer-Anschluss als Verbindungssystem für Leitungen im medizinischen Bereich, insbesondere für Fluid-, Unterdruck- und Druckluftleitungen, mit einem ersten männlichen Verbindungselement, das einen distalen Anschlussbereich aufweist und einem zweiten weiblichen Verbindungselement, das einen distalen Aufnahmebereich aufweist, wobei der Anschlussbereich in den Aufnahmebereich einsteckbar ist, so dass eine feste Verbindung zwischen dem männlichen Verbindungselement und dem weiblichen Verbindungselement geschaffen ist.

Verbindungssysteme der gattungsbildenden Art sind seit Jahren aus der Praxis bekannt und werden beispielsweise zur Verbindung von Schlauchsystemen verwendet. Dies können Infusionsschläuche aber beispielsweise auch Versorgungsschläuche sein, die vom Operateur benötigte Handgeräte mit Druckluft, pulsierender Druckluft, Unterdruck, Flüssigkeit, etc. versorgen. Beispielsweise in der Ophthalmologie dienen diese zum Absaugen von Flüssigkeit im Rahmen der Entfernung des Linsenkörpers nach dessen Zertrümmerung.

Diese sogenannte Luer-Verbindung besteht aus einem weiblichen und einem männlichen Luer-Anschluss. Der männliche Luer-Anschluss weist ein kegelförmig ausgebildetes Endstück auf. Dieses ist in die ebenfalls kegelförmige Öffnung des weiblichen Luer-Anschlusses einbringbar. Drückt man das Endstück des männlichen Luer-Anschlusses in die Öffnung des weiblichen Luer-Anschlusses, wird eine feste Verbindung geschaffen.

Das gattungsbildende Verbindungssystem ist problematisch, da beliebige Verbindungselemente miteinander verbunden werden können. Wird beispielsweise während eines chirurgischen Eingriffs aus Unachtsamkeit der Anschluss eines zum Absaugen dienenden Handgeräts mit einem Druckluft liefernden Schlauch verbunden, so kann dies zu erheblichen Verletzungen führen. Auch bei der Vergabe von Medikamenten über eine Infusion kann die Verbindung des Infusionsschlauchs mit einem "falschen" Infusionsbeutel für den Patentien mitunter gefährliche Auswirkungen haben.

Ein weiteres Problem liegt darin, dass zwei miteinander verbundene Leitungen sich sehr leicht wieder trennen lassen, wenn der weibliche und der männliche Luer-Anschluss mit zu geringer Kraft ineinander gepresst wurden. Beispielsweise während eines chirurgischen Eingriffs kann durch eine Zugbelastung an einer der Leitungen die Verbindung versehentlich gelöst werden.

Beispielsweise aus den Dokumenten WO 2008/009946 A1, EP 1 902 747 A2, GB 2 451 891 A, FR 2 924 616 A1, US 2008/0318456 A1 und US 2006/0033331 A1 sind jeweils Verbindungssysteme für Leitungen im medizinischen Bereich bzw. Luer-Verbindungen vorbekannt.

Die GB 2 379 253 A zeigt des Weiteren ein Verbindungssystem bestehend aus einem männlichen Verbindungselement 16 und einem weiblichen Verbindungselement 1. Das weibliche Verbindungselement 1 greift dabei über ein Außengewinde in ein korrespondierendes Innengewinde 19 des männlichen Verbindungselements 16 ein.

Der vorliegenden Erfindung liegt daher die Aufgabe zugrunde, ein gattungsbildendes Verbindungssystem für Leitungen im medizinischen Bereich derart auszugestalten und weiterzubilden, dass nur geeignete Verbindungselemente miteinander verbunden werden können, wobei eine feste Verbindung durch konstruktiv einfache Mitteln gewährleistet sein soll.

Die voranstehende Aufgabe ist erfindungsgemäß durch ein Verbindungssystem für Leitungen im medizinischen Bereich mit den Merkmalen des Patentanspruchs 1 gelöst. Danach ist das gattungsbildende Verbindungssystem für Leitungen im medizinischen Bereich dadurch gekennzeichnet, dass die Nuten im Endbereich verjüngt ausgebildet sind.

Erfindungsgemäß ist erkannt worden, dass die Verbindung nicht geeigneter Verbindungselemente durch eine Kodierung der Verbindungselemente in überraschend einfacher Weise verhindert werden kann. Dazu weist der Anschlussbereich eine Kodierungsschelle auf, so dass die Verbindung mit einem weiblichen Verbindungselement nur dann möglich ist, wenn dieses eine mit der Kodierungsschelle korrespondierende Kodierungsoberfläche aufweist. Die Verbindung mit einem weiblichen Luer-Anschluss, wie er aus dem Stand der Technik bekannt ist, ist durch die Kodierungsschelle sicher verhindert. Es lassen sich nur solche Verbindungselemente miteinander verbinden, die die gleiche Kodierung tragen. Der Benutzer kann sich stets sicher sein, dass nur geeignete Verbindungselemente miteinander verbunden sind.

Durch die Kodierungsschelle findet eine weitere Klemmung zwischen den zu verbindenden Anschlüssen statt, nämlich zwischen der Oberfläche des Aufnahmebereichs und der Innenseite der Kodierungsschelle. Durch diese konstruktive Maßnahme ist eine sichere Verbindung gewährleistet, auch wenn die Verbindungselemente vom Anwender nur mit geringer Kraft ineinander gedrückt wurden. Ein versehentliches Lösen der Verbindung ist damit weitestgehend ausgeschlossen ist.

Die Kodierungsschelle ist in vorteilhafter Weise zylinderförmig ausgebildet und kann mindestens bis zum distalen Ende des Anschlussbereichs des männlichen Verbindungselements verlaufen. Der Anschlussbereich ist dadurch bis zu seinem distalen Ende hin von der Kodierungsschelle umgeben.

In weiter erfindungsgemäßer Weise weist die Kodierungsschelle an ihrer Innenseite mindestens ein Kodierungselement auf. Das Kodierungselement kann sich derart zur Außenseite des männlichen Verbindungselements erstrecken, dass ein gattungsbildendes weibliches Verbindungselement nicht zwischen Kodierungsschelle und männlichem Verbindungselement einbringbar ist.

Das Kodierungselement ist als Rippe bzw. Strebe ausgebildet. Das Einbringen des Anschlussbereichs des männlichen Verbindungselements in den Aufnahmebereich des weiblichen Verbindungselements ist nur dann möglich, wenn der Aufnahmebereich eine mit dem Kodierungselement korrespondierende Kodierungsoberfläche aufweist. Das versehentliche Verbinden nicht geeigneter Verbindungselemente ist somit nicht möglich. Die Kodierungsrippen sind derart ausgelegt, dass sie im verbundenen Zustand auf die Oberfläche des Aufnahmebereichs gepresst werden. Durch diese Konstruktion ist das weibliche Verbindungselement auf der Innenseite mit dem männlichen Verbindungselement und auf der Außenseite mit der Kodierungsschelle verklemmt. Dies gewährleistet eine äußerst effektive und sichere Verbindung.

Um die Griffigkeit der Kodierungsschelle zu erhöhen, kann diese an ihrer Außenseite eine strukturierte Oberfläche aufweisen. Diese kann beispielsweise in Form von Rillen, Riefen oder Streben ausgebildet sein. Der Verwender kann somit beim Verbinden bzw. Lösen der Verbindungselemente an der Kodierungsschelle angreifen, so dass die Handhabung - insbesondere beim Lösen einer festen Verbindung - vereinfacht ist.

Die Kodierungsschelle kann gegenüber dem männlichen Verbindungselement unbeweglich ausgeführt sein. In vorteilhafter Weise ist die Kodierungsschelle als integraler Bestandteil des männlichen Verbindungselements vorgesehen.

In besonders vorteilhafter Weise ist die Kodierungsschelle gegenüber dem männlichen Verbindungselement beweglich. Die Oberfläche des männlichen Verbindungselements kann beispielsweise eine Wulst oder eine lineare Vertiefung aufweisen, an der die Kodierungsschelle befestigbar ist. Hierdurch ergibt sich der Vorteil, dass die Kodierungsschelle beim Verbinden der Verbindungselemente derart drehbar ist, dass die Kodierungsrippen passend zur korrespondierenden Kodierungsoberfläche ausgerichtet werden. Die Kodierungsrippen können derart ausgebildet sein, dass sie eine Pilotfunktion erfüllen, um die Kodierungsschelle richtig auszurichten. Hierzu können sich die Kodierungsrippen in ihrem distalen Endbereich beispielsweise verjüngen. Die Verbindungselemente müssen somit beim Verbinden nicht gegeneinander gedreht werden. Dies verhindert ein versehentliches Verdrehen bzw. Abknicken der Leitungen beim Ineinanderstecken der Verbindungselemente.

Die Kodierungsoberfläche ist durch Nuten, Streben oder Rillen gebildet. Diese sind vorteilhafter Weise derart ausgelegt, dass sie gleitend mit den Kodierungsrippen in Verbindung treten. Ein weiterer Vorteil liegt darin, dass der Aufnahmebereich durch die Nuten, Streben oder Rillen verstärkt ist. Um die Handhabung für den Anwender weiter zu vereinfachen, können zueinander passende männliche und weibliche Verbindungselemente beispielsweise eine einheitliche farbliche Markierung aufweisen.

In besonders vorteilhafter Weise ist an dem den Aufnahmebereich gegenüberliegenden Endbereich des weiblichen Verbindungselements eine Leitung vorgesehen. Dies kann beispielsweise ein Fluid-, Unterdruck- oder Druckluftschlauch sein. Die Leitungen können beispielsweise adhäsiv mit dem weiblichen Verbindungselement verbunden sein.

Um zu verhindern, dass beispielsweise Schläuche über den Endbereich des weiblichen Verbindungselements gezogen werden, kann dieser eine strukturierte Oberfläche aufweisen. Ein über den Endbereich gezogener Schlauch kann aufgrund einer derart ausgestalteten Oberfläche nicht druckfest an das weibliche Verbindungselement gekoppelt werden. Die strukturierte Oberfläche kann beispielsweise durch Rillen, Riefen oder Streben gebildet sein. Ein weiterer Vorteil liegt darin, dass die Handhabung des weiblichen Verbindungselements durch die griffigere Oberfläche erheblich vereinfacht ist.

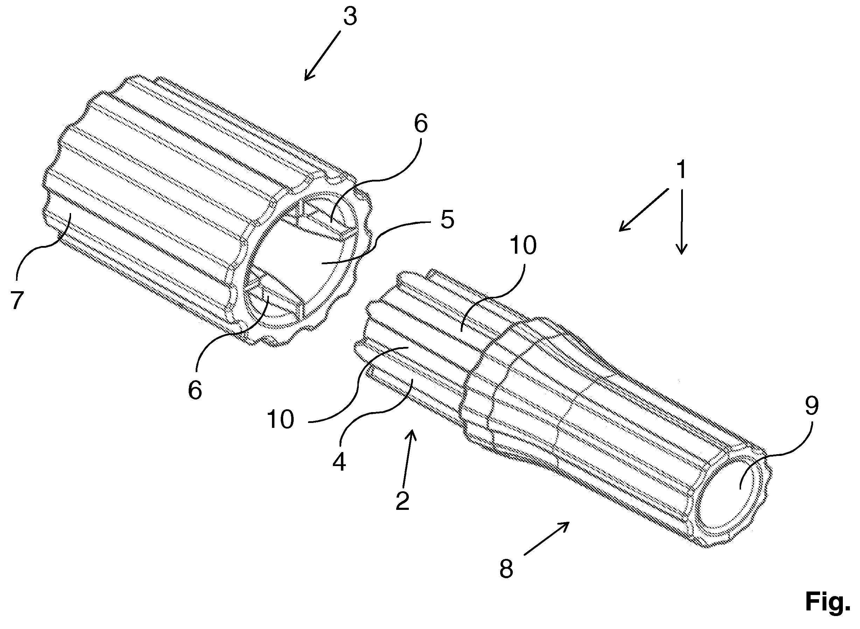

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigt die
- einzige Fig.: in einer schematischen Darstellung ein Ausführungsbeispiel einer erfindungsgemäßen Kodierungsschelle und eines erfindungsgemäßen weiblichen Verbindungselements.

Die einzige Fig. zeigt ein Ausführungsbeispiel einer erfindungsgemäßen Kodierungsschelle 3 sowie eines erfindungsgemäßen weiblichen Verbindungselements 1.

Die Kodierungsschelle 3 umschließt das nicht dargestellte erste männliche Verbindungselement. Die Kodierungsschelle 3 weist an ihrer Innenseite 5 zwei Kodierungselemente 6 auf. Die Kodierungselemente 6 sind als Kodierungsrippen ausgeführt. An ihrem distalen Ende sind die Kodierungselemente 6 radial nach außen geneigt, so dass sie einfacher über den Aufnahmebereich 2 des weiblichen Verbindungselements 1 geschoben werden können. Die Außenseite 7 der Kodierungsschelle 3 ist strukturiert ausgeführt, um die Griffigkeit für den Anwender zu erhöhen.

Der Aufnahmebereich 2 des weiblichen Verbindungselements 1 weist die Kodierungsoberfläche 4 auf. An der Kodierungsoberfläche 4 sind mehrere Nuten 10 vorgesehen, in die die Kodierungsrippen 6 eingreifen. Im Endbereich verjüngen sich die Nuten 10, um eine Einführhilfe für die Kodierungsrippen 6 zu bilden. Durch diese Einführhilfe wird die Kodierungsschelle 3 beim Verbinden automatisch derart gedreht, dass die Kodierungselemente 6 passend zu den Nuten 10 ausgerichtet werden.

Im Endbereich 8 des weiblichen Verbindungselements 1 ist eine Öffnung 9 vorgesehen, in die ein nicht dargestellter Schlauch eingebracht und verklebt wird. Um zu verhindern, dass ein Schlauch über den Endbereich 8 gezogen werden kann weist der Endbereich 8 eine strukturierte Oberfläche auf. Somit kann keine dichte Verbindung zwischen dem Endbereich 8 und einem darüber gezogenen Schlauch geschaffen werden. Des Weiteren erhöht die strukturierte Oberfläche des Endbereichs 8 die Griffigkeit des weiblichen Verbindungselements 1 und vereinfacht dadurch die Handhabung.

Im verbundenen Zustand liegt das männliche Verbindungselement im Innern des Aufnahmebereichs 2 des weiblichen Verbindungselements 1. Der Aufnahmebereich 2 ist von der Kodierungsschelle 3 umschlossen, wobei die Kodierungselemente 6 fest auf der Kodierungsoberfläche 4 aufliegen.

Hinsichtlich weiterer vorteilhafter Ausgestaltungen des erfindungsgemäßen Verbindungssystems wird zur Vermeidung von Wiederholungen auf den allgemeinen Teil der Beschreibung sowie auf die Patentansprüche verwiesen.

Schließlich sei ausdrücklich darauf hingewiesen, dass das voranstehend beschriebene Ausführungsbeispiel des erfindungsgemäßen Verbindungssystems lediglich zur Erörterung der beanspruchten Lehre dient, diese jedoch nicht auf das Ausführungsbeispiel einschränkt.

### Bezugszeichenliste

- 1: weibliches Verbindungselement
- 2: distaler Aufnahmebereich
- 3: Kodierungsschelle
- 4: Kodierungsoberfläche
- 5: Innenseite/Kodierungsschelle
- 6: Kodierungselement
- 7: Außenseite/Kodierungselement
- 8: Endbereich
- 9: Öffnung
- 10: Nut

## Patentansprüche

1. Luer-Anschluss als Verbindungssystem für Leitungen im medizinischen Bereich, insbesondere für Fluid-, Unterdruck- und Druckluftleitungen, mit einem ersten männlichen Verbindungselement, das einen distalen Anschlussbereich aufweist und einem zweiten weiblichen Verbindungselement (1), das einen distalen Aufnahmebereich (2) aufweist, wobei der Anschlussbereich in den Aufnahmebereich (2) einsteckbar ist, so dass eine feste Verbindung zwischen dem männlichen Verbindungselement und dem weiblichen Verbindungselement (1) geschaffen ist, wobei der Anschlussbereich eine Kodierungsschelle (3) und der Aufnahmebereich (2) eine mit der Kodierungsschelle (3) korrespondierende Kodierungsoberfläche (4) aufweist, wobei die Kodierungsschelle (3) an ihrer Innenseite (5) mindestens ein als Rippe ausgebildetes Kodierungselement (6) aufweist, dass die Kodierungsoberfläche (4) durch Nuten (10) gebildet ist, dass die Rippe (6) im verbundenen Zustand auf die Oberfläche des Aufnahmebereichs (2) gepresst ist, so dass das weibliche Verbindungselement (1) auf der Innenseite mit dem männlichen Verbindungselement und auf der Außenseite mit der Kodierungsschelle (3) verklemmt ist,
**dadurch gekennzeichnet, dass** die Nuten (10) im Endbereich verjüngt ausgebildet sind.

2. Luer-Anschluss nach Anspruch 1, **dadurch gekennzeichnet, dass** die Kodierungsschelle (3) zylinderförmig ausgebildet ist.

3. Luer-Anschluss nach Anspruch 1 oder 2, **dadurch gekennzeichnet, dass** die Kodierungsschelle (3) an ihrer Außenseite (7) eine strukturierte Oberfläche, insbesondere Rillen, Riefen oder Streben, aufweist.

4. Luer-Anschluss nach einem der Ansprüche 1 bis 3, **dadurch gekennzeichnet, dass** die Kodierungsschelle (3) gegenüber dem männlichen Verbindungselement unbeweglich, vorzugsweise integraler Bestandteil des männlichen Verbindungselements ist.

5. Luer-Anschluss nach einem der Ansprüche 1 bis 3, **dadurch gekennzeichnet, dass** die Kodierungsschelle (3) gegenüber dem männlichen Verbindungselement beweglich ist.

6. Luer-Anschluss nach einem der Ansprüche 1 bis 5, **dadurch gekennzeichnet, dass** das weibliche Verbindungselement (1) an seinem dem Aufnahmebereich (2) gegenüberliegenden Endbereich (8) eine Leitung, beispielsweise eine Fluidleitung oder Unterdruckleitung oder Druckluftleitung, aufweist.

7. Luer-Anschluss nach Anspruch 5, **dadurch gekennzeichnet, dass** die Leitung mit dem weiblichen Verbindungselement (1) adhäsiv verbunden ist.

8. Luer-Anschluss nach einem der Ansprüche 1 bis 7, **dadurch gekennzeichnet, dass** der Endbereich (8) eine strukturierte Oberfläche, insbesondere Rillen, Riefen oder Streben, aufweist.

## Claims

1. Luer connection as a connecting system for lines in the medical sector,
in particular for fluid, vacuum and compressed air lines, with a first male connecting element that has a distal connecting region and a second female connecting element (1) that has a distal receiving region (2), wherein the connecting region can be inserted into the receiving region (2), so that a strong connection is made between the male connecting element and the female connecting element (1), wherein the connecting region has an encoding collar (3) and the receiving region (2) has an encoding surface (4) corresponding to the encoding collar (3), wherein the encoding collar (3) has on its inner side (5) at least one encoding element (6) formed as a rib, that the encoding surface (4) is formed by grooves (10), that, in the connected state, the rib (6) is pressed on to the surface of the receiving region (2) such that the female connecting element (1) is jammed on the inner side with the male connecting element and on the outer side with the encoding collar (3),
c h a r a c t e r i s e d in that the grooves (10) are tapered in the end region.

2. Luer connection according to claim 1, **characterised in that** the encoding collar (3) is cylindrical in shape.

3. Luer connection according to claim 1 or 2, **characterised in that** the encoding collar (3) has a structured surface, in particular flutes, grooves or stays on its outer side (7).

4. Luer connection according to one of the claims 1 to 3, **characterised in that** the encoding collar (3) is immovable with respect to the male connecting element, preferably being an integral component of the male connecting element.

5. Luer connection according to one of the claims 1 to 3, **characterised in that** the encoding collar (3) can move with respect to the male connecting element.

6. Luer connection according to one of the claims 1 to 5, **characterised in that** the female connecting element (1) has a line, for example a fluid line or vacuum line or compressed air line, on its end region (8) opposite the receiving region (2).

7. Luer connection according to claim 5, **characterised in that** the line is connected to the female connecting element (1) by adhesive.

8. Luer connection according to one of the claims 1 to 7, **characterised in that** the end region (8) has a structured surface, in particular flutes, grooves or stays.

## Revendications

1. Raccord luer en tant que système de raccordement pour des conduites dans le domaine médical, en particulier pour des conduites de fluide, de vide et d'air comprimé, avec un premier élément de raccordement mâle qui présente une zone de raccord distale, et avec un deuxième élément de raccordement femelle (1) qui présente une zone de réception (2) distale, la zone de raccord pouvant être enfichée dans la zone de réception (2) de telle sorte qu'un raccordement ferme est créé entre l'élément de raccordement mâle et l'élément de raccordement femelle (1), la zone de raccord présentant une bride de codage (3), et la zone de réception (2) présentant une surface de codage (4) correspondant à la bride de codage (3), la bride de codage (3) présentant sur son côté intérieur (5) au moins un élément de codage (6) constitué en tant que nervure, que la surface de codage (4) est formée de rainures (10), que la nervure (6) est, dans l'état raccordé, pressée sur la surface de la zone de réception (2) de telle sorte que l'élément de raccordement femelle (1) est coincé sur le côté intérieur avec l'élément de raccordement mâle, et sur le côté extérieur avec la bride de codage (3), **caractérisé en ce que** les rainures (10) sont constituées en se rétrécissant dans la zone d'extrémité.

2. Raccord luer selon la revendication 1, **caractérisé en ce que** la bride de codage (3) est constituée en forme de cylindre.

3. Raccord luer selon la revendication 1 ou 2, **caractérisé en ce que**, sur son côté extérieur (7), la bride de codage (3) présente une surface structurée, en particulier des cannelures, des rainures ou des nervures.

4. Raccord luer selon l'une des revendications 1 à 3, **caractérisé en ce que**, par rapport à l'élément de raccordement mâle, la bride de codage (3) est immobile, de préférence elle fait partie intégrante de l'élément de raccordement mâle.

5. Raccord luer selon l'une des revendications 1 à 3, **caractérisé en ce que** la bride de codage (3) est mobile par rapport à l'élément de raccordement mâle.

6. Raccord luer selon l'une des revendications 1 à 5, **caractérisé en ce que** l'élément de raccordement femelle (1) présente, à sa zone d'extrémité (8) faisant face à la zone de réception (2), une conduite, par exemple une conduite de fluide ou de vide ou d'air comprimé.

7. Raccord luer selon la revendication 5, **caractérisé en ce que** la conduite est raccordée de façon adhésive à l'élément de raccordement femelle (1).

8. Raccord luer selon l'une des revendications 1 à 7, **caractérisé en ce que** la zone d'extrémité (8) présente une surface structurée, en particulier des cannelures, des rainures ou des nervures.
